(19) Europäisches Patentamt
European Patent Office
Office européen des brevets

(11) Publication number: **0 369 092 B1**

(12) # EUROPEAN PATENT SPECIFICATION

(45) Date of publication of patent specification: **22.12.93**

(51) Int. Cl.5: **A61L 15/58**, C09J 133/06

(21) Application number: **89102340.0**

(22) Date of filing: **10.02.89**

The file contains technical information submitted after the application was filed and not included in this specification

(54) **Dermal pressure-sensitive adhesive sheet material.**

(30) Priority: **17.11.88 JP 290651/88**

(43) Date of publication of application:
**23.05.90 Bulletin 90/21**

(45) Publication of the grant of the patent:
**22.12.93 Bulletin 93/51**

(84) Designated Contracting States:
**DE FR GB SE**

(56) References cited:
**EP-A- 0 107 915**
**EP-A- 0 123 465**
**EP-A- 0 174 803**
**WO-A-88/01877**
**GB-A- 1 473 972**
**US-A- 4 608 249**

(73) Proprietor: **NITTO DENKO CORPORATION**
**1-2, Shimohozumi 1-chome**
**Ibaraki-shi**
**Osaka(JP)**

(72) Inventor: **Sugii, Tetsuji**
**NITTO DENKO CORPORATION**
**1-2, Shimohozumi 1-chome**
**Ibaraki-shi Osaka(JP)**
Inventor: **Wada, Shintaro**
**NITTO DENKO CORPORATION**
**1-2, Shimohozumi 1-chome**
**Ibaraki-shi Osaka(JP)**
Inventor: **Konno, Masayuki**
**NITTO DENKO CORPORATION**
**1-2, Shimohozumi 1-chome**
**Ibaraki-shi Osaka(JP)**

(74) Representative: **Patentanwälte Grünecker, Kinkeldey, Stockmair & Partner**
**Maximilianstrasse 58**
**D-80538 München (DE)**



Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services
(3.10/3.6/3.3.1)

## Description

The present invention relates to a dermal pressure-sensitive adhesive sheet material for, for example, an adhesive bandage, surgical elements, an adhesive plaster, a dressing and a drape.

Conventional dermal pressure-sensitive adhesive sheet materials such as an adhesive bandage, comprise a backing having provided on one surface thereof a pressure-sensitive adhesive layer. These sheet materials are used by adhering the same to the skin by means of an adhesive force of the adhesive layer.

The backing in the conventional dermal pressure-sensitive adhesive sheet materials usually comprises polyethylene or polyester. Therefore, these materials do not have a low modulus of elasticity behavior similar to a skin, and cannot follow expansion and contraction of the skin. As a result, a patient feels a cramp on the skin as he moves. Thus, the use feeling thereof is uncomfortable.

Further, because those materials do not have a sufficient moisture-permeability, sweat is gathered between the skin and the pressure-sensitive adhesive layer to increase the water content of the epidermis more than necessary, which may injure the normal skin. The sweat gathered also tends to reduce the adhesive force of the pressure-sensitive adhesive layer to cause slippage or peel of the sheet material off the skin.

In order to overcome the disadvantages attributed to poor moisture-permeability, it is suggested to make the pressure-sensitive adhesive sheet porous or finely porous. However, such porous sheets are not satisfactory from the standpoint of sanitation because it is feared that water or bacteria may enter through pores to reach the surface of the skin.

Further, the pressure-sensitive adhesive which contacts the skin of the human body generally has a strong irritation to the skin, and long-term contact with the skin induces itching and, if the skin is irritable, inflammations or erruptions may occur.

In addition, since the skin has an irregular and complicated surface, it has been very difficult to develop an adhesive having good adhesive properties to the skin combined with a satisfactory cohesive force.

From US-A-4608249 a patching layer containing a drug is known which comprises 15 to 85% by weight of an alkyl acrylate or alkyl methacrylate, 5 to 75% by weight of a (meth)acrylic ester having an ether group and 10 to 50% by weight of a copolymerisable polymer monomer. This patching layer has a good adhesiveness to the skin as well as good sweat-removing properties.

The object underlying the present invention is to provide a dermal pressure-sensitive adhesive sheet material which causes no irritation to the skin, has a low elasticity behavior similar to a skin and exhibits high moisture-permeability, excellent bacteria resistance, and excellent water resistance.

In accordance with the present invention the above object is attained with a dermal pressure-sensitive adhesive sheet material which comprises a backing having provided on one side thereof a pressure-sensitive adhesive layer, wherein the pressure-sensitive adhesive layer contains a copolymer comprising from 40 to 80% by weight of an alkyl acrylate or methacrylate monomer, from 10 to 50% by weight of an alkoxyl-containing ethylenically unsaturated monomer, and from 1 to 10% by weight of a carboxyl-containing ethylenically unsaturated monomer and having a glass transition point of 250 °K or lower and a gel fraction after drying of 25% or more, provided that the amount of the carboxyl-containing ethylenically unsaturated monomer is not 10% by weight.

Figure 1 is a cross section of the dermal pressure-sensitive adhesive sheet material according to the present invention.

Figure 2 is an illustration of the pressure-sensitive adhesive sheet material of the present invention as applied to the skin.

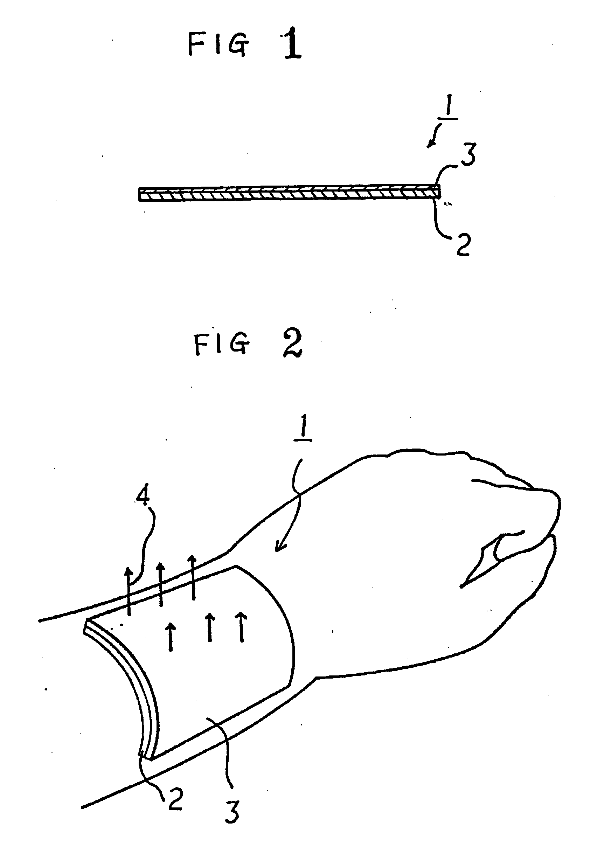

Referring to Fig. 1, the dermal pressure-sensitive adhesive sheet material (1) of the present invention comprises a backing (2) having provided on one side thereof a pressure-sensitive adhesive layer (3).

The reasons for using the alkyl acrylate or methacrylate monomer are that the monomer is a component which imparts adhesive properties to the pressure-sensitive adhesive, which does not irritate the skin, which has an excellent transparency, and which makes it difficult to cause deterioration of the adhesive properties even when used over a long period of time.

In the present invention, the alkyl acrylate or methacrylate must be used in an amount of 40 to 80% by weight based on the weight of the copolymer. The reason is explained below.

If the amount of the alkyl acrylate or methacrylate monomer in the copolymer is less than 40% by weight, sufficient adhesive properties cannot be obtained. If it exceeds 80% by weight, the desired cohesive force cannot be obtained.

Specific examples of the alkyl acrylate or methacrylate monomer are butyl acrylate, propyl acrylate, isooctyl acrylate, isononyl acrylate, 2-ethylhexyl acrylate, decyl acrylate and the corresponding

methacrylates. These monomers may be used either individually or in combination of two or more thereof.

The alkoxyl-containing ethylenically unsaturated monomer is a component that imparts a moisture-permeability to the adhesive. Further, after drying the copolymer, it undergoes a de-alcohol reaction with the carboxyl-containing monomer to form a crosslinked structure.

The alkoxyl-containing ethylenically unsaturated monomer must be used in an amount of 10 to 50% by weight. The reason is described below.

If the amount of the alkoxyl-containing monomer is less than 10% by weight, the moisture-permeability and gel fraction are too small to attain the desired cohesive force. It it exceeds 50% by weight, the copolymer becomes too hydrophilic to exhibit sufficient adhesive properties to the skin.

Specific examples of the alkoxyl-containing ethylenically unsaturated monomer are methoxypolyethylene glycol, methoxypolyethylene glycol methacrylate, ethoxydiethylene glycol acrylate, butoxydiethylene glycol acrylate, methoxyethyl acrylate, methoxyethyl methacrylate, 3-ethoxypropyl acrylate, ethoxyethyl acrylate, ethoxyethyl methacrylate, butoxyethyl acrylate, and butoxyethyl methacrylate. Those monomers may be used either individually or in combination of two or more thereof.

The carboxyl-containing ethylenically unsaturated monomer functions to improve the cohesion of the adhesive, and also serves as a reactant in the crosslinking reaction with the alkoxyl-containing monomer.

Specific examples of the carboxyl-containing monomer are acrylic acid, methacrylic acid, itaconic acid, and maleic acid.

The carboxyl-containing ethylenically unsaturated monomer must be used in an amount of 1 to 10% by weight provided that 10% by weight is excluded. The reason therefor is described below.

If the amount of the carboxyl-containing monomer is less than 1% by weight, the cohesive force becomes relatively weak. On the other hand, if it exceeds 10% by weight, not only does the cohesive force become excessively high, but the many polar groups introduced into the copolymer make the adhesive irritative, causing erruptions on the skin when adhered.

In the preparation of the dermal pressure-sensitive adhesive sheet of the present invention, a general polymerization catalyst, such as azobis compounds and peroxide compounds, can be used. The polymerization can be carried out by any technique, such as solution polymerization, emulsion polymerization, and suspension polymerization. For example, a monomer mixture comprising the above-described monomers, a solvent, e.g., ethyl acetate, and a radical generating catalyst are charged in a polymerization vessel, and polymerization is completed in a period of from 10 to 20 h.

If desired, the monomer mixture may further contain a vinyl monomer.

It is required that the resulting copolymer has a glass transition point of 250°K or lower and a gel fraction after drying of 25% or more. If the glass transition point exceeds 250°K, the desired adhesive properties and cohesive force cannot be attained. If the gel fraction after drying is less than 25%, the desired cohesive force cannot be obtained.

Formation of a gel on drying can be accounted for by formation of a crosslinked structure by using the above-described monomers. In general, when a crosslinked structure is formed, the adhesive properties are reduced. In the present invention, however, it was confirmed that the reduction in adhesive properties is minimized as compared with the increase of the cohesive force due to the crosslinked structure.

The thickness of the pressure-sensitive adhesive layer is not particularly limited but preferably ranges from 10 to 50 µm. A thickness of less than 10 µm tends to have insufficient adhesive properties to the skin, while a thickness exceeding 50 µm tends to have an insufficient moisture-permeability. A suitable thickness can be determined according to the portion of the skin to which the adhesive layer is adhered.

Materials of the backing which can be used in the present invention include polyether urethane, polyester urethane, polyether-polyamide block copolymers, and polyacrylate. Preferred materials are polyether-polyamide block copolymers represented by formula:

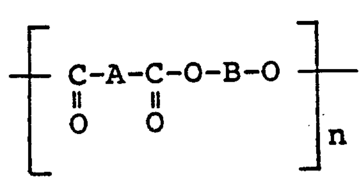

wherein A represents a polyamide component selected from nylon 6, nylon 6.6, nylon 10, nylon 11, and nylon 12; B represents a polyether component selected from polyethylene glycol, polypropylene glycol, and polytetramethylene glycol; and n represents a positive integer.

The polyamide component as represented by A can be obtained by polycondensation between a dicarboxylic acid (e.g., terephthalic acid, oxalic acid, adipic acid, sebasic acid, 1-cyclohexydicarboxylic acid) and a diamine (e.g., ethylenediamine, pentamethylenediamine, hexamethylenediamine), polymerization of cyclic lactams (e.g., captrolactam, caprylolactam, caprinlactam, laurinlactam), polycondensation of aminocarboxylic acids, or copolymerization of the above-described cyclic lactams, dicarboxylic acids, and diamines.

The polyether component as represented by B includes polyoxyalkylene glycols, e.g., polyoxyethylene glycol, polyoxypropylene glycol, and polyoxytetramethylene glycol.

A commercially available product (e.g., "PEBAX"® ) may be utilized as the above-described block copolymer.

The thickness of the backing is not particularly limited, but preferably ranges from 20 to 100 µm. A too thin backing is inconvenient on handling such as adhering or peeling. A too thick backing may lose its softness and interfere with the moisture-permeation. A proper thickness of the backing can be determined according to the portion of the skin to which the backing is adhered.

When the adhesive sheet material of the present invention is applied to parts having a relatively large expansion and contraction of the skin, such as joints, it is preferred that the backing has a tensile strength of from 100 to 500 $kg/cm^2$ and a 100% modulus of from 10 to 100 $kg/cm^2$. If the tensile strength exceeds 500 $kg/cm^2$ or the 100% modulus exceeds 100 $kg/cm^2$, it is difficult to impart a softness to the backing, and when it is adhered on the skin as a dermal pressure-sensitive adhesive sheet material, the sheet material cannot follow the expansion and contraction of the skin, thereby causing physical irritations on the skin and an uncomfortable feeling, or slippage or peel of the sheet material. On the other hand, if the tensile strength is less than 100 $kg/cm^2$ or 100% modulus is less than 10 $kg/cm^2$, the base sheet is too soft to handle. Thus, problems on use occur.

The block polymer represented by the above-described formula can be made to have the tensile strength and modulus in the above-described ranges by changing the ratio of a hard segment and a soft segment in the block polymer. That is, it is preferred that the weight percentage of the polyether component B is 50 to 90% by weight based on the total weight of the polyamide component A which is the hard segment component and the polyether component B which is the soft segment component.

However, in the event that the dermal pressure-sensitive adhesive sheet material of the present invention is applied to the skin of parts making relatively small movements, such as, e.g., thighs, legs, arms and chest, the tensile strength and 100% modulus of the backing are not particularly restricted and can be appropriately selected.

When the dermal pressure-sensitive adhesive sheet material of the present invention is applied to parts which are easily wet with sweat, such as the palm of the hand and the sole of the foot, it is preferred that the adhesive layer has a moisture-permeability of 1,000 $g/m^2$ or more during 24 h at 40 °C and that the laminate of the backing and the adhesive layer as a whole has a moisture-permeability of 800 $g/m^2$ or more during 24 h at 40 °C.

If the moisture-permeability of the pressure-sensitive adhesive layer is less than 1,000 $g/m^2$ during 24 h at 40 °C or the moisture-permeability as a whole of the laminate of backing and pressure-sensitive adhesive layer is less than 800 $g/m^2$ during 24 h at 40 °C, there is a tendency that sweat is gathered between the pressure-sensitive adhesive layer and the skin surface to increase the water content of the epidermis more than necessary, which may cause injury of the normal skin, or a reduction of the adhesive force of the pressure-sensitive adhesive to cause slippage or peel.

When the dermal pressure-sensitive adhesive sheet material is applied to parts having a relatively small sweating, the moisture-permeability of the pressure-sensitive adhesive layer and the dermal pressure-sensitive adhesive sheet material as a whole is not limited to the above-described range and is appropriately selected.

The pressure-sensitive sheet according to the present invention can be prepared by directly coating the pressure-sensitive adhesive on a backing, drying the adhesive, and applying a release sheet on the adhesive layer; or coating the pressure-sensitive adhesive on a release sheet, drying the adhesive, and adhering a backing to the adhesive layer. By this procedure, a dermal pressure-sensitive adhesive sheet with the pressure-sensitive adhesive layer protected with the release sheet can be obtained.

The structure of the dermal pressure-sensitive adhesive sheet material according to the present invention is as described above. In using the dermal pressure-sensitive adhesive sheet material, as shown in Fig. 2, the adhesive sheet material of the present invention can be adhered to the skin of the affected part utilizing the adhesive force of the pressure-sensitive adhesive layer. The dermal pressure-sensitive adhesive sheet thus adhered can easily follow the movement of the skin, while removing sweat as moisture (4).

The dermal pressure-sensitive adhesive sheet material according to the present invention exhibits the following effects.

The adhesive is non-irritative to the skin and excellent in transparency, and the adhesion thereof is not reduced during long-term use. Further, since the copolymer has a glass transition point of 250 °K or less and a gel fraction after drying of 25% or more, a satisfactory adhesive property and cohesive force can be obtained, which assure excellent holding properties particularly when the adhesive sheet material is used as an element for fixing medical equipment. Thus, the adhesive sheet material causes no strong irritation to the skin or suffers from no reduction in adhesion during long-term use and therefore is excellent in safety and stability.

In particular, when the moisture-permeability of the pressure-sensitive adhesive layer is 1,000 g/$m^2$ or more and that of the laminate of the backing and the adhesive layer is 800 g/$m^2$ or more, the sweat from the skin is effectively vaporized without being collected between the skin and the pressure-sensitive adhesive layer so that there is no fear that the water content of the epidermis excessively increases to injure the normal skin or that the adhesion of the adhesive layer is reduced to cause slippage or peel.

Since no pores for moisture escape are required, water or bacteria are not allowed to reach the skin. This also means that the exudate from the affected part would not ooze of the backing. Namely, the adhesive sheet is very sanitary.

In particular when the backing has a tensile strength of from 100 to 500 kg/$cm^2$ and a 100% modulus of from 10 to 100 kg/$cm^2$, the similarity to the skin in elasticity is further ensured, while retaining easiness on handling such as adhereing or peeling, and the backing can further securely follow the movement of the skin.

The present invention is now illustrated in greater detail by reference to Examples and Comparative Examples. In these examples, all the parts are by weight unless otherwise indicated.

The copolymers and pressure-adhesive sheets prepared in the Examples and Comparative Examples were evaluated for physical properties or performance properties according to the following test methods.

1) Adhesive Strength of the Adhesive Sheet Material:

A 20 mm wide dermal pressure-sensitive adhesive sheet material was subjected to a peel test at a peel angle of 180° using a Bakelite plate as an adherend according to JIS Z-1528 to measure the adhesive strength (g/20 mm).

2) Holding Time of the Adhesive Sheet:

A dermal pressure-sensitive adhesive sheet material having a size of 10 mm x 20 mm was adhered onto a Bakelite plate. A load of 500 g was applied thereon at 40 °C, and the time (min) until the adhesive sheet fell off the plate was measured. The adhesive sheet material under test was backed with a polyester film to prevent stretching of the sheet material during the load application.

3) Moisture-Permeability of the Adhesive Sheet Material:

10 ml of distilled water were put in a glass-made container having an inner diameter of 38 mm and a height of 40 mm, and an adhesive sheet material having a diameter of 50 mm was placed thereon and fixed with its adhesive layer facing downward to cover the opening in. The container was allowed to stand in an air-conditioned room at 40 °C and 30% RH for 24 h, and the loss of water in the container was measured to calculate the moisture permeability (g/$m^2$ during 24 h at 40 °C).

4) Gel Fraction of the Copolymer:

A predetermined amount of a dried polymer was extracted with ethyl acetate for 24 h, and the residue was weighed. The gel fraction was calculated from formula:

$$\frac{\text{(Weight of Extraction Residue)}}{\text{(Weight of Polymer Before Extraction)}} \times 100\ (\%)$$

EXAMPLE 1

| | |
|---|---|
| 2-Ethylhexyl acrylate | 70 parts |
| Ethoxyethyl acrylate | 25 parts |
| Acrylic acid | 5 parts |
| Ethyl acetate | 150 parts |
| Azoisobutyronitrile | 0.3 part |

The above components were charged in a polymerization vessel, and the mixture was stirred while displacing the atmosphere with nitrogen. Thereafter, polymerization was conducted for about 10 h while keeping the inner temperature at 55 to 65 °C. The inner temperature was raised up to 70 °C, at which the stirring was continued for an additional about 2 h. The resulting copolymer had a glass transition point (Tg) of 212 °K and a gel fraction of 31.5%.

The copolymer was coated on release paper having been treated with silicone to a dry thickness of 25 µm and dried at 130 °C for 5 min to form a pressure-sensitive adhesive layer. A 35 µm thick polyether-polyamide block copolymer sheet ("PEBAX 3533"®), one side of which had been subjected to a corona discharge treatement, was adhered onto the adhesive layer under pressure to obtain a dermal pressure-sensitive adhesive sheet material.

The adhesive strength, holding time, and moisture-permeability of the resulting adhesive sheet material are shown in Table 1.

EXAMPLE 2

A pressure-sensitive adhesive sheet material was prepared in the same manner as in Example 1, except for using the following monomer mixture. The Tg and gel content of the copolymer and the adhesive strength, holding time, and moisture-permeability of the adhesive sheet material are shown in Table 1.

| | |
|---|---|
| Isononyl acrylate | 62 parts |
| Methoxyethyl acrylate | 35 parts |
| Acrylic acid | 3 parts |

EXAMPLE 3

A pressure-sensitive adhesive sheet material was prepared in the same manner as in Example 1, except for using the following monomer mixture. The Tg and gel fraction of the copolymer and the adhesive strength, holding time, and moisture-permeability of the adhesive sheet material are shown in Table 1.

| | |
|---|---|
| 2-Ethylhexyl acrylate | 65 parts |
| Butoxyethyl acrylate | 30 parts |
| Methacrylic acid | 5 parts |

COMPARATIVE EXAMPLE 1

A pressure-sensitive adhesive sheet was prepared in the same manner as in Example 1, except for replacing ethyl acetate with a mixed solvent of toluene and laurylcaptane (100/0.025 by weight). The Tg and gel fraction of the copolymer and the adhesive strength, holding time, and moisture-permeability of the adhesive sheet material are shown in Table 1.

COMPARATIVE EXAMPLE 2

A pressure-sensitive adhesive sheet material was prepared in the same manner as in Example 1, except for changing the drying condition to 95 °C and 5 min. The Tg and gel fraction of the copolymer and

the adhesive strength, holding time, and moisture-permeability of the adhesive sheet material are shown in Table 1.

COMPARATIVE EXAMPLE 3

A pressure-sensitive adhesive sheet material was prepared in the same manner as in Example 1, except for using the following monomer mixture. The Tg and gel fraction of the copolymer and the adhesive strength, holding time, and moisture-permeability of the adhesive sheet material are shown in Table 1.

| | |
|---|---|
| 2-Ethylhexyl acrylate | 95 parts |
| Acrylic acid | 5 parts |

TABLE 1

| Example No. | Adhesive Strength (g/19 mm) | Holding Time | Moisture Permeability (g/m·24 hr·40°C) | Tg (°C) | Gel Fraction (%) |
|---|---|---|---|---|---|
| Example 1 | 420 | 100 min or more | 950 | 212 | 31.5 |
| " 2 | 500 | " | 1100 | 207 | 28.0 |
| " 3 | 450 | " | 880 | 214 | 30.5 |
| Comparative Example 1 | 490 | fall in 30 min (cohesive failure) | 1050 | 212 | 0 |
| " 2 | 400 | fall in 15 min (cohesive failure) | 950 | 212 | 5 |
| " 3 | 500 | fall in 30 min (cohesive failure) | 530 | 208 | 0 |

**Claims**

1. A dermal pressure-sensitive adhesive sheet material which comprises a backing having provided on one side thereof a pressure-sensitive adhesive layer, wherein said pressure-sensitive adhesive layer contains a copolymer comprising from 40 to 80% by weight of an alkyl acrylate or methacrylate monomer, from 10 to 50% by weight of an alkoxyl-containing ethylenically unsaturated monomer, and from 1 to 10% by weight of a carboxyl-containing ethylenically unsaturated monomer and having a glass transition point of 250°K or lower and a gel fraction after drying of 25% or more, provided that the amount of the carboxyl-containing ethylenically unsaturated monomer is not 10% by weight.

2. A dermal pressure-sensitive adhesive sheet material as claimed in claim 1, wherein said pressure-sensitive adhesive layer has a moisture-permeability of 1,000 $g/m^2$ or more during 24h at 40°C and said adhesive sheet material has a moisture-permeability of 800 $g/m^2$ or more during 24h at 40°C.

3. A dermal pressure-sensitive adhesive sheet material as claimed in claim 1, wherein said backing has a tensile strength of from 100 to 500 $kg/cm^2$ and a 1009 modulus of from 10 to 100 $kg/cm^2$.

4. A dermal pressure-sensitive adhesive sheet material as claimed in claim 1, wherein said backing comprises a polyether-polyamide block copolyer represented by formula

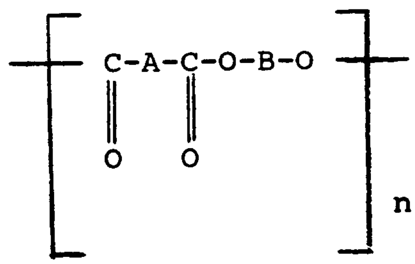

wherein A represents a polyamide component selected from the group consisting of nylon 6, nylon 6.6, nylon 10, nylon 11 and nylon 12; B represents a polyether component selected from the group consisting of polyethylene glycol, polypropylene glycol and polytetramethylene glycol; and n represents a positive integer.

## Patentansprüche

1. Dermales, druckempfindliches Klebefolienmaterial, welches einen Träger mit einer auf einer Seite davon vorgesehenen druckempfindlichen Klebschicht umfaßt, wobei die druckempfindliche Klebschicht ein Copolymer enthält, das 40 bis 80 Gew.-% eines Alkylacrylat- oder -methacrylatmonomers, 10 bis 50 Gew.-% eines Alkoxyl-haltigen ethylenisch-ungesättigten Monomers und 1 bis 10 Gew.-% eines Carboxy-haltigen ethylenischungesättigten Monomers umfaßt und eine Glasübergangstemperatur von 250 ° K oder weniger und einen Gelanteil nach dem Trocknen von 25 % oder mehr aufweist, mit der Maßgabe, daß die Menge des Carboxy-haltigen ethylenisch-ungesättigten Monomers nicht 10 Gew.-% ist.

2. Dermales, druckempfindliches Klebefolienmaterial nach Anspruch 1, wobei die druckempfindliche Klebschicht eine Feuchtedurchlässigkeit von 1000 g/$m^2$ oder mehr während 24 h bei 40 ° C aufweist, und das Klebefolienmaterial eine Feuchtedurchlässigkeit von 800 g/$m^2$ oder mehr während 24 h bei 40 ° C aufweist.

3. Dermales, druckempfindliches Klebefolienmaterial nach Anspruch 1, worin der Träger eine Zugfestigkeit von 100 bis 500 kg/$cm^2$ und einen 100 %-Modul von 10 bis 100 kg/$cm^2$ aufweist.

4. Dermales, druckempfindliches Klebefolienmaterial nach Anspruch 1, worin der Träger ein Polyether-Polyamid-Blockcopolymer umfaßt, das durch die Formel

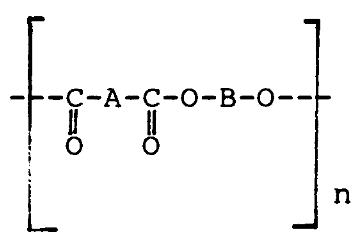

dargestellt ist, worin A eine Polyamidkomponente, ausgewählt aus der Gruppe, bestehend aus Nylon 6, Nylon 6,6, Nylon 10, Nylon 11 und Nylon 12, darstellt; B eine Polyetherkomponente, ausgewählt aus der Gruppe, bestehend aus Polyethylenglykol, Polypropylenglykol und Polytetramethylenglykol, darstellt; und n eine positive ganze Zahl darstellt.

## Revendications

1. Feuille dermique revêtue d'un adhésif sensible à la pression, qui comprend un support comportant sur une de ses faces une couche d'adhésif sensible à la pression, ladite couche d'adhésif sensible à la pression contenant un copolymère comprenant de 40 à 80 % en poids d'un acrylate ou méthacrylate d'alkyle monomère, de 10 à 50% en poids d'un monomère à insaturation éthylénique contenant un groupe alcoxyle et de 1 à 10 % en poids d'un monomère à insaturation éthylénique comprenant un groupe carboxyle et ayant un point de transition vitreuse égal ou inférieur à 250 ° K et une action de gel, après séchage, égale ou supérieure à 25 %, à la condition que la quantité du monomère à insaturation éthylénique contenant un groupe carboxyle ne soit pas égale à 10 % en poids.

2. Feuille dermique revêtue d'un adhésif sensible à la pression, telle que revendiquée à la revendication 1, dans laquelle ladite couche adhésive sensible à la pression a une perméabilité à l'humidité égale ou supérieure à 1 000 $g/m^2$ en 24 h à 40 ° C et ladite feuille revêtue de l'adhésif présente une perméabilité à l'humidité égale ou supérieure à 800 $g/m^2$ en 24 h à 40 ° C.

3. Feuille dermique revêtue d'un adhésif sensible à la pression, telle que revendiquée à la revendication 1, dans le cas de laquelle ledit support a une résistance à la traction de 100 à 500 $kg/cm^2$ et un module à 100 % de 10 à 100 $kg/cm^2$.

4. Feuille dermique revêtue d'un adhésif sensible à la pression, telle que revendiquée à la revendication 1, dans laquelle ledit support comprend un copolymère à blocs ou longues séquences polyéther-polyamide, représenté par la formule :

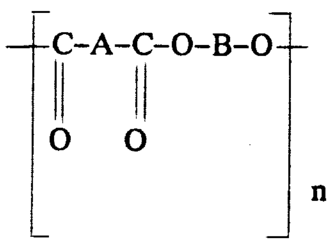

dans laquelle A représente un constituant polyamide choisi dans l'ensemble consistant en du "Nylon 6", du "Nylon 6.6", du "Nylon 10", du "Nylon 11" et du "Nylon 12" ; B représente un constituant polyéther choisi dans l'ensemble consistant en du polyéthylèneglycol, du polypropylèneglycol et du polytétraméthylèneglycol ; et n représente un nombre entier positif.

FIG 1

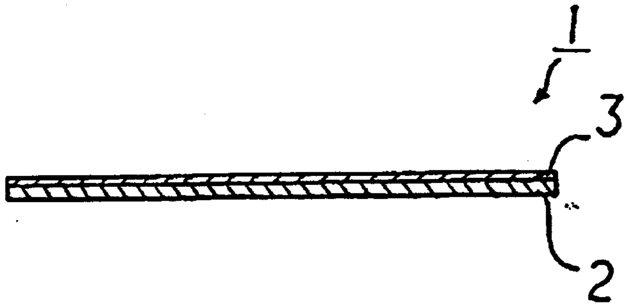


FIG 2